# EUROPEAN PATENT SPECIFICATION

(11) **EP 3 772 358 B1**
(45) Date of publication and mention of the grant of the patent: **14.09.2022**
(21) Application number: 19190550.4
(22) Date of filing: 07.08.2019
(51) Int. Cl.: A61M 5/142, A61B 5/145

(54) **MEDICAL DEVICE WITH TACTILE INDICATION**
MEDIZINISCHE VORRICHTUNG MIT TAKTILER ANZEIGE
DISPOSITIF MÉDICAL À INDICATION TACTILE

(43) Date of publication of application: 10.02.2021
(73) Proprietor: Roche Diabetes Care GmbH, 68305 Mannheim (DE); F. Hoffmann-La Roche AG, 4070 Basel (CH)
(72) Inventor: KONYA, Ahmet, 68305 Mannheim (DE)
(74) Representative: Pfaffmann, Lucas

(56) References cited:
- EP-A1- 2 009 533
- CN-B- 105 788 189
- US-A1- 2012 277 668

## Description

### FIELD OF THE INVENTION

The present invention relates to medical systems that comprise a body-mountable medical device with an activatable tactile indicator, as well as to methods for providing tactile indications to a user of such medical system. The invention is particularly useful in the context of diabetes therapy, but may also be used in other diagnostic and/or therapeutically applications.

### BACKGROUND OF THE INVENTION

Medical devices, in particular therapeutic and/or diagnostic devices that are carried by a patient substantially continuously are used in increasing numbers in various therapies. By way of example it is an established form of therapy in the treatment of diabetes mellitus to supply insulin in a substantially continuous manner, night and day, by way of a miniaturized computer-controlled insulin infusion pump and a subcutaneous infusion cannula (Continuous Subcutaneous Insulin Infusion, CSII). Classically, such insulin infusion pumps have a shape and size similar to a cigarette package and are carried, e. g. in trousers' pockets, with a belt clip, or the like. Here, the insulin infusion pump is connected with a subcutaneous cannula via tubing. Cannula, tubing and an insulin cartridge are replaced by the patient (also referred to as user in the following) himself/herself as required, e. g. every few days.

Over the last years, body-mountable infusion pumps, e. g. insulin infusion pumps, have been developed that are typically somewhat smaller and are designed and shaped to be directly attached to the patient's skin respectively body either directly or via a cradle for a continuous application in a typical range of days to weeks. Those body-mountable insulin infusion pumps are preferred by many patients mainly for discreetness reasons and are further advantageous regarding a number of technical and performance aspects. Further, continuous glucose measurement systems are increasingly used in diabetes-therapy to enable substantially continuous monitoring and control of the blood glucose. Those continuous glucose measurement devices may also be body-mountable as explained before and have a transcutaneous electrochemical sensing element. Throughout the present document, the expression "body-mountable" refers to devices that are designed to be firmly but releasable attached, typically adhesively attached, to the skin for a generally extend application time either directly or via a cradle, as opposed to devices that are designed to be carried in a trousers' pocket, with a belt clip, as necklace, or the like and to be connected to the body only via a (normally flexible) electric and/or fluidic line, such as a cable and/or tubing.

Medical devices and systems such as continuous glucose measurement devices or insulin infusion pumps are typically designed to provide information to the user. Complex information is typically provided to the user on a display that may be part of the device itself or of a remote device, for example a hand-held remote controller. In addition, however, they are typically designed to provide audible and/or tactile indications, similar to a cell phone or pager device. Tactile indication, in particular by way of vibration, is known to be preferred by many users over audible indications for discreetness reasons. Information that is typically provided in this way is feedback in reaction to a user operation, as well as - even more important - the alerting in situations that should be brought to the user's attention and may require immediate action, for example an empty battery, a depleted drug cartridge, a blocked infusion cannula, or a device defect. A device or component that is designed to provide tactile indication is throughout this document referred to as "tactile indicator". Prior art devices with tactile indicators are disclosed in US 2012/277668 A1, EP 2009533 A1 and CN 105788189.

### SUMMARY OF THE INVENTION

When providing information by way of a tactile indication, in particular vibration, phantom vibrations may occur, meaning that a user believes that the tactile indicator has been activated to provide a tactile indication, while this is actually not the case. The occurrence of phantom vibrations is at least annoying for many users. Further, it is observed that under certain circumstances an actual activation of the tactile indicator is not recognized by the user. While in any case being undesirable, such scenario is potentially even more critical and dangerous, since it may result in a situation that requires direct attention being recognized by the user too late or not at all.

In principle, phenomenon of phantom vibrations as well as unrecognized vibrations may be avoided by making the vibration very strong. This, however, is undesirable for a number of reasons such as discerns, device size, and power consumption. Also, it is known to provide an acoustic indication in addition to or alternatively to a tactile indication in critical situations, in particular if a first tactile indication is not acknowledged by the user within a predefined time window. While such additional acoustic indication may be unavoidable and even required under certain circumstances, it is generally undesirable since it contradicts the desired discreteness.

It is an overall objective to improve the state of the art regarding tactile indications to a user of a body-mountable medical device and favorably reduce or avoid some or all of the before-mentioned problems.

In an aspect, the overall objective is achieved by a medical system. The medical system includes a skin-mountable medical device, the skin-mountable medical device including an activatable tactile indicator. The medical system further includes a user interface. The user interface is configured to receive location information, the location information indicating a body location where the skin-mountable medical device is mounted or shall be mounted. The medical system is further configured to determine an active control pattern in dependence of the location information and to control activation of the tactile indicator in accordance with the active control pattern. The active control pattern is also referred to as "selected control pattern".

In a further aspect, the overall objective is achieved by a method of providing tactile indications to a user of a medical system, wherein the medical system includes a skin-mountable medical device. The method includes (a) providing, via a user interface of the medical system, location information. The location information indicating a body location where the skin-mountable medical device is mounted or shall be mounted. The location information is received via the user interface. The method further includes determining, by the medical system, an active control pattern in dependence of the location information. The method further includes controlling activation of the activatable tactile indicator in accordance with the active control pattern. The expression "tactile indicator" and "tactile indication" refer to indications and indicators that are in a first case and preliminary tactile, while other kinds of sensory perceptions, in particular audible perception, are at least secondary and favorably negligible or not present at all. "Acoustic indication", to the contrary refers to an indication that is mainly or exclusively acoustical. By being mounted onto a patient's skin upon activation of the tactile indicator the tactile indication directly stimulates the haptic sense of the patient. This is for instance advantageous in a noisy environment, in which acoustic signals may be covered by background noise or a patient's clothing.

According to the invention, the tactile indicator is activated respectively controlled differently in dependence of the body location where the body-mountable medical device is carried. This allows a tuning of the tactile indication in dependence of the body location, thereby reducing and favorably eliminating the before-described phenomena of phantom vibrations and unrecognized vibrations.

In dependence of the overall design of the user interface, the user interface may be designed to receive location information in various ways. By way of example, the user interface may provide alternative body locations where the body-mountable medical device may be carried by a list from which a selection is made by the user for example by way of clicking a checkbox or the like. In another example with the graphical user interface (GUI), the user interface may show a (typically schematic) body and a selection is made by the user by way of selecting the corresponding body location, for example using a mouse or a touch screen. In further embodiments, a default body location may be suggested by the medical device on the user interface. The default body location may be confirmed or altered by the user. By way of example, the medical system may be designed to suggest default body locations according to a user-defined or factory-set sequential order for repeated applications of the body-mountable medical device. Typically, the body-mountable medical device is carried at specific body location for a limited period of time in the range of a number of days to a number of weeks. Subsequently, the body-mountable medical device is removed and re-mounted at the different body location. In dependence of the type of medical device, this process may be carried out along with further maintenance steps such as replacing a transcutaneous sensor, a drug cartridge, and/or an infusion cannula. Typical body locations for carrying body-mountable medical devices include different regions of the abdomen, the upper arms, the thighs, and the buttocks.

The medical system may in some embodiments be realized as a single body-mountable compact device, with the user interface being realized with the body-mountable medical device in an integral way. In other embodiments the user interface is provided on a separate device, also referred to as remote device. The overall objective underlying the present invention is achieved irrespective of which entity of the medical system determines and/or is used for selecting and/or determining an active control pattern in dependence of the location information indicating a body location where the skin-mountable medical device is or shall be mounted.

Consequently, in a further aspect, the overall objective is achieved by a remote device including a user interface. The user interface is configured to receive location information indicating a body location where a skin-mountable device including an activatable tactile indicator is mounted or shall be mounted. The remote device is further configured to determine an active control pattern in dependence of the location information and i) to control activation of the tactile indicator of the skin-mountable device in accordance with the active control pattern and/or ii) for transmitting information indicative of the active control pattern from the remote device to the skin-mountable device.

The remote device, thus, can be used to select and/or to determine an active control pattern for controlling the activatable tactile indicator of a skin-mountable device in dependence on the location information received via the user interface. The remote device can be configured to actually control the activatable tactile indicator or to transmit information indicative of the control pattern from the remote device to the skin-mountable device. In the latter case, it is intended that the skin-mountable medical device is configured to control activation of the tactile indicator in accordance with the active control pattern indicated by the received information.

In an embodiment, the activatable tactile indicator is a vibrator. The term "vibrator" is to be understood as an electrically actuated device that generates a mechanical vibration upon actuation. Vibrators within this meaning are also commonly used, e. g., in cell phones or pager devices.

In some embodiments with a vibrator, the vibrator is a coin vibration motor. Generally, vibration motors are based on the rotation of an eccentric mass coupled to the motor shaft causing vibration due to the unbalanced mass. A coin vibration motor (also referred to as flat vibration motor or pancake vibration motor) comprises of a particularly thin, coin-shaped motor. In some further embodiments with a vibrator, the vibrator is an eccentric rotating mass vibration motor (ERM). For this type of design, an eccentric mass is mounted to the axis of a miniaturized electric motor of typical cylindrical shape. In still some further embodiments with a vibrator, the vibrator is a linear resonant actuator (LRA). Such LRAs are generally designed similar to a dynamic loudspeaker as known in the art, but are pro-vided with a vibration mass instead of a membrane. Their overall shape may be coin-like as for a coin vibration motor. In an embodiment the vibrator comprises a piezoelectric actuator. In still further embodiments, the tactile indicator comprises an electro-stimulation device supplementary or instead of a mechanically vibrating device. In some embodiments the tactile indicator does not generate (mechanical) vibrations, but is an electro-stimulation device. In still further embodiments, the tactile indicator includes one or more tactile stimulation elements, such as pins, that are actuable (for example by way of electromagnets) to slightly press into the skin.

In an embodiment, the activatable tactile indication is a vibrator and different control patterns among a set of predetermined control patterns for operating the activatable tactile indicator differ in at least one of frequency, amplitude, number of activation bursts and sequences of activation bursts, and pauses between activation bursts. Generally, the tactile indicator is activated by providing a control signal, for example a DC or AC voltage. Control patterns which differ in at least one of frequency and amplitude accordingly differ in the frequency and/or amplitude of the control signal. Parameters of the control signal that are used for controlling the tactile indicator are also referred to as "control parameters". Frequency and amplitude are accordingly control parameters. Generally, different control patterns differ in at least one control parameter, in particular frequency, amplitude, number of activation bursts, sequences of activation bursts, and pauses between activation bursts. In dependence of the overall design and in particular the used tactile indicator, further control parameters may be used additionally and/or alternatively, for example a waveform of the control signal. The waveform is particularly suited as control signal in embodiments where the control signal is an AC signal. The control parameters that are associated with the active control pattern are also referred to as "active control parameters".

In an embodiment, the medical system comprises a set of predetermined control patterns and the active control pattern is selected from the set of predetermined control patterns.

A method for providing tactile indications may include selecting the active control pattern from a set of predetermined control patterns included by the medical system. For instance, the set of predetermined control patterns can be stored in a memory of the medical system. Storing control patterns is to be understood as storing the corresponding control parameters as explained before. In accordance with the location information, the corresponding control pattern is recalled.

In an embodiment, a correspondence between body locations and control patterns is factory-set. For this type of embodiment, a control pattern is pre-defined and included by the medical system for each body location. One control pattern may be pre-defined and included for each body location. In a variant, a number of more than one available control patterns is pre-defined and stored for some or all body locations. In such embodiment, the medical system may be configured to execute and the method may include a preparatory step of assigning either of the available control patterns to each body location based on user input, e. g. via the user interface. This assignment may be done by patient who carries the medical system and/or another user or operator, such as a healthcare professional. This preparatory step typically needs to be carried out when taking into operation a new medical system and/or medical device. It generally does not need to be carried out every time the body-wearable medical device is placed at a different body location.

In a further embodiment, available control patterns are user-defined. In such embodiment, the medical system may be configured, in the preparatory step, to automatically vary one or more control parameters while actuating the tactile indicator, thereby tuning one or more control parameters. Via the user interface, the medical system may receive a user input when the tactile indication is considered optimal. The corresponding setting of control parameter or control parameters may be stored by the medical system as control pattern. The body location may be inputted by the user. The preparatory step may be carried out for a specific body location, for example when carrying the body-mountable medical device at a new body location for the first time, and/or may be carried out for a number of body locations in sequence. This may be the case, for example, when putting a new medical system and/or body-mountable medical device into operation.

In an embodiment, the medical system includes a remote device, wherein the skin-mountable medical device and the remote device are configured for wireless data exchange with each other, wherein the user interface is provided on the remote device. A method for providing tactile indications may include providing the user interface on a remote device.

The remote device may be a portable device, such as a handheld remote control device for the body mountable medical device. In further embodiments, the remote device is a general-purpose device, such as a smart phone, that runs corresponding software code respectively stores a corresponding application that enables the general-purpose device, upon executing the software code respectively running the application, to act as remote device. In still further embodiments, the remote device may be a non-portable or limited portable device, such as a personal computer or laptop computer with the corresponding software.

In an embodiment with a remote device, the remote device is configured to determine the active control pattern and the medical system is configured for transmitting information indicative of the active control pattern from the remote device to the skin-mountable medical device.

In this type of embodiment, the requirements at the site of the body-mountable medical device are comparatively low since the selection of the control pattern is carried out on the remote device and only the information regarding this control pattern needs to be stored by the body-mountable medical device. The information that is transmitted from the remote device to the body-mountable medical device may directly include some or all control parameters, such as amplitude and/or frequency as explained above. Alternatively, the information that is transmitted may simply be identification information, such as a number, with the actual control parameters being stored by the body-mountable medical device.

In a further embodiment with a remote device, the medical system is configured for transmitting information indicative of the location information from the remote device to the skin-mountable medical device and the skin-mountable medical device is configured to determine the active control pattern based on the retrieved information. In comparison to the before-described embodiments, additional hardware and/or software effort is required on the side of the body-mountable medical device for this type of embodiment, while the requirements the site of the remote device are lower.

In an embodiment, the skin-mountable medical device includes memory configured to store active control parameters for the tactile indicator. The active control parameters are associated with the active control pattern. The body-mountable medical device is configured to control activation of the tactile indicator according to the active control parameters. As explained before, the memory may, in dependence of the embodiment, also store non-active control parameters that are associated with available but (currently) non-active control patterns.

In an embodiment, the skin-mountable medical device includes at least one of a continuous glucose measurement device and an insulin infusion pump. Both continuous glucose measurement devices and into infusion pumps are typically used in the therapy of diabetes mellitus and are generally widely known in the art. The continuous glucose measurement device typically operates on an electrochemical principle and comprises the transcutaneous or implantable sensor element with one or more electrodes and corresponding control, signal processing and/or evaluation circuitry. An insulin infusion pump is typically (but not necessarily) a positive displacement pump, e. g. a syringe driver pump, and is designed to infuse insulin over according in a continuous or substantially continuous (quasi-continuous) manner according to a time-variable basal infusion regime and is further designed to administer insulin boli on demand.

In an embodiment, the medical system is configured to control activation of the activatable tactile indicator in accordance with an indication information that shall be indicated to the patient. A method for providing tactile indications may include controlling activation of the activatable tactile indicator in accordance with an indication information that shall be indicated to the patient. Generally, any kind of information that is provided by the tactile indicator is referred to as "indication information". The indication information may include user-feedback, statues information, such as the content of an insulin cartridge or a battery state, alerts regarding situation that should be brought to the user's attention, for example a close-to-depleted battery or close-to-empty insulin cartridge, as well as warnings and alerts in case of situations that require user's immediate action, such as a completely emptied insulin cartridge, a blocked infusion cannula, or a device malfunction. Controlling activation of the tactile indicator in accordance with an indication information may, for example, include switching the tactile indicator on and off according to a pre-defined indication pattern. To the extent that a range or variety of control parameters as explained before is equally suited to provide tactile indications as at a specific body location, control parameters may also be set in accordance with the body location. In such embodiment, control parameters, such as amplitude, frequency or waveform may depend on both the body location and the indication information.

It is evident for a person skilled in the art that features of the remote device which are described herein in the context of a medical system are also regarded to be disclosed for the remote device on its known. Likewise, features of the medical system which are described herein in the context of a remote device are also regarded to be disclosed for the medical system on its known. In particular, features of the medical system which are described in the context of method of providing tactile indications are also regarded to be disclosed for the medical system on its known. Features of the method of providing tactile indications which are described in the context of the medical system are also regarded to be disclosed for the method of providing tactile indications on its known.

### BRIEF DESCRIPTION OF THE DRAWINGS

- Fig. 1: schematically shows a medical system in accordance with the present disclosure;
- Fig. 2: schematically shows the indications of body locations on a graphical user interface;
- Fig. 3: schematically shows the storing of control patterns and control parameters;
- Fig. 4: schematically shows different tactile indications.

### EXEMPLARY EMBODIMENTS

In the following, exemplary embodiments are explained in more detail with additional reference to the figures.

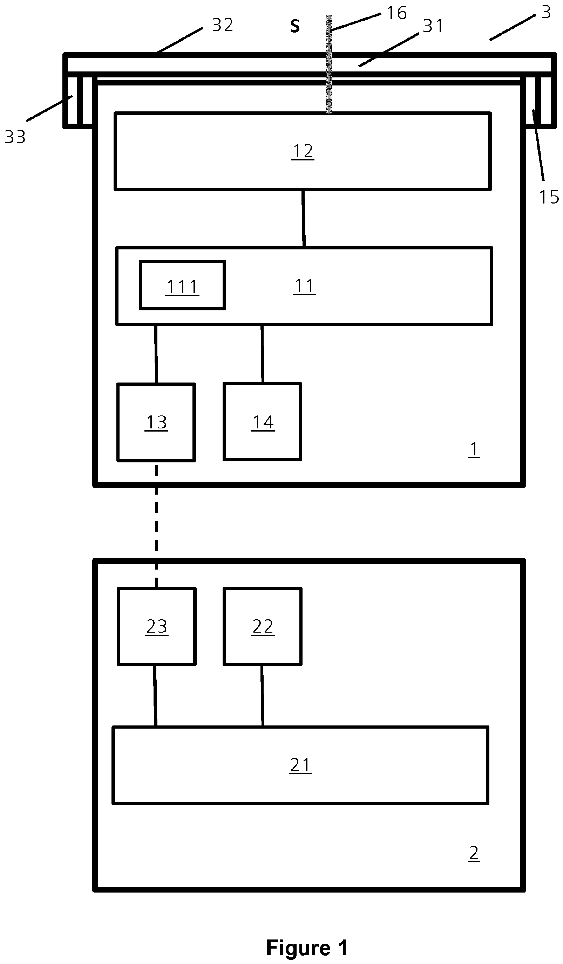

**Figure 1** shows an exemplary medical system in a schematic view. In this example, the medical system includes the skin-mountable medical device 1, and a remote device 2. The skin-mountable device may be temporarily mounted to a patient's body via a cradle 3. By way of example, the skin-mountable medical device 1 is an insulin infusion pump with functionality as explained in the general description. Further by way of example, the remote device 2 is a portable handheld device. The remote device 2 may in particular be a smart phone or a dedicated remote control device for the skin-mountable medical device 1.

The skin-mountable medical device 1 includes a medical device control unit 11, a functional medical unit 12, a wireless medical device communication unit 13, and a tactile indicator 14.

The medical device control unit 11 serves the purpose of controlling the overall-operation of the skin-mountable medical device 1. The medical device control unit 11 may include one or more programmable circuits, such as microprocessors and/or microcontrollers, ASICs, or the like, and the corresponding programming respectively software or firmware code. Additionally, or alternatively, the medical device control unit 11 may include special-purpose circuitry.

The functional medical unit 12 comprises the diagnostic and/or therapeutically and in general medical functionality of the skin-mountable medical device 1. In case of the medical device 1 being an insulin infusion pump as assumed here, the functional medical unit 12 is or includes a pump unit. In an alternative embodiment where the skin-mountable medical device 1 is a continuous glucose measurement device, the functional medical unit 12 may be or include a glucose measurement unit. In the shown example, a transcutaneous element 16 projects from the functional medical unit 12 into the skin S or is part of the functional medical unit. In case of the functional medical unit 12 being a pump unit, the transcutaneous element 16 may be an infusion cannula. In case of the functional medical unit 12 being a glucose measurement unit, the transcutaneous element may be an electrochemical sensing element. In further embodiments, a sensing element may be fully implanted and communicate with the body-mountable wearable device transcutaneous e. g. via a wireless near field communication link.

The wireless medical device communication unit 13 is typically (but not necessarily) based on RF technology, for example Bluetooth or other standard or proprietary communication protocols and technics.

By way of example, the tactile indicator 14 is a linear resonant actuator (LRA). In further similar embodiments, the tactile indicator 14 may for example be motor-based vibrator as also typically used in cell phone or pager devices and as explained in the general description above.

The operation of the functional medical unit 12, the medical device communication unit 13 and the tactile indicator 14 as well as of potential further units or components of the ambulatory medical device 1 is coordinated and controlled by the medical device control unit 11. Therefore, the medical device control unit 11 is operatively coupled with the functional medical unit 12, the medical device communication unit 13 and the tactile indicator 14.

The medical device control unit 11 further includes memory 111 that stores one or more control patterns as explained further below. It is to be understood that the medical device control unit 111 may comprise further volatile and/or non-volatile memory (not explicitly shown) that stores program code and further data as generally known in the art. Such further memory may be integral with memory 111.

For the purpose of skin-attaching respectively skin-mounting the skin-mountable medical device 1, the medical system comprises in this example cradle 3 as structurally distinct additional component. The cradle 3 has a cradle body 31 that is substantially plate-shaped and may be substantially planar. The cradle body 31 has a proximal side with an adhesive layer 32 for attaching respectively mounting to the skin S. At an opposed distal side (pointing away from the patient's body during application), a medical device coupling structure 33 is present. The medical device coupling structure 33 is designed for engagement with a cradle coupling structure 15 of the skin-mountable medical device 1. In application, the medical device coupling structure 33 and the cradle coupling structure 15 form a mechanical coupling via positive locking and/or force looking. By way of example, the medical device coupling structure 33 and the cradle coupling structure 15 may, in combination, form a latch-catch structure. In dependence of the overall design and the lifetime of the skin-mountable medical device 1, the coupling between the skin-mountable medical device 1 and the cradle 3 may be releasable or non-releasable. It is further noted that the presence of a cradle 3 is not essential. In alternative embodiments, the skin-facing surface of the skin-mountable medical device 1 carries the adhesive layer 32, thereby allowing the skin-mountable medical device 1 to be directly attached to the skin S.

The remote device 2 includes a remote device control unit 11, a wireless remote device communication unit 23 and a user interface 22.

The remote device control unit 21 may be realized in a similar manner as explained before with respect to the medical device control unit 11 and may be include programmable multi-purpose components running corresponding code respectively programming and/or special-purpose circuitry. The remote device control unit 21 controls overall operation of the remote device 2.

The remote device communication unit 23 may be designed in the same way as the medical device communication unit 13. The medical device communication unit 13 and the remote device communication unit 23 are designed for data exchange with each other. The data that may be exchanged include data related to tactile indications as explained further below.

The remote device user interface 22 is in this example realized as graphical user interface (GUI) with a touchscreen that serves for both receiving respectively inputting and providing respectively outputting data from/to a user. Those data may in particular be related to tactile indications. It is noted, however, that the design of the user interface 22 is not essential and could be realised in different way too.

The operation of the remote device communication unit 23 and the user interface 22 as well as of potential further units or components of the remote device 2 is coordinated and controlled by the remote device control unit 21. Therefore, the remote device control unit 21 is operatively coupled with the remote device communication unit 23 and the user interface 22.

It is noted that the shown structure of both the body-mountable medical device 1 and the remote device 2 serves the purpose of illustration. In fact, a number or all elements or units may be realized in a fully or partly integral way.

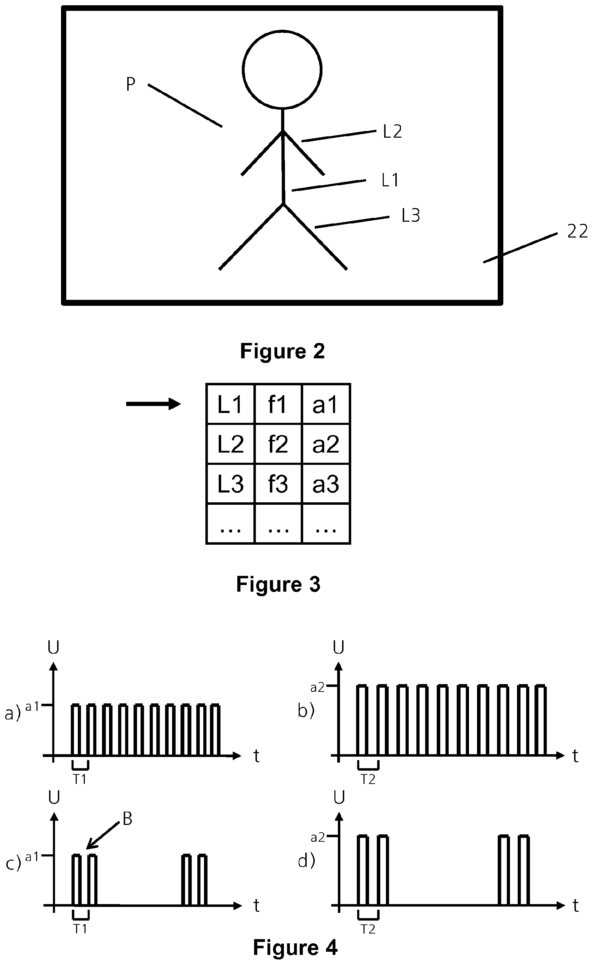

**Figure 2** schematically shows an exemplary display on the touchscreen respectively GUI 22. For inputting location information, a schematic representation P of the patient's body is shown with an indication of body locations where the body-mountable medical device 1 may be mounted respectively attached. By way of example, the body locations are the abdomen, L1, the upper arms, L2, and the thighs, L3. Before or subsequent to attaching the body-mountable device 1, L1, the user selects the body location by touching the corresponding portion of the body representation P. As mentioned before, other ways of inputting the body location may be used as well. Information indicative of the selected body location, for example a code representing the selected body location, is transmitted to the body-mountable medical device 1 via the communication units 13, 23. By way of example, it is assumed that the abdomen, L1, is selected.

**Figure 3** schematically shows an example of storing control parameters for a number of available control patterns in the memory 111. Memory 11 stores in this example a table where each row corresponds to a specific body location L1, L2, L3, ... and an associated available control pattern. Further, a column is provided for each control parameter of the control patterns. By way of example, the control parameters are the frequency and the amplitude for activating the tactile indicator 14, number of activation bursts, sequences of activation bursts, and pauses between activation bursts. The selection of L1 as body location (indicated by an arrow) accordingly results in an activation of the tactile indicator 14 with frequency f1 and amplitude a2 for providing tactile indications under control of the medical device control unit 11. In related embodiments with other type of tactile indicators, other control parameters and in particular more or less control parameters may be provided. By way of example, if the tactile indicator 14 is based on a standard DC motor as actuator, the voltage for powering the monitor may be the sole control parameter. It is here exemplarily assumed that the control parameters for the available control patterns are pre-stored, e. g. as factory settings, in the memory 111.

Further it is noted that the information as shown in Figure 3 may alternatively be stored in the remote device 2 rather than the body-mountable medical device 1, and the selected control parameters, e. g. a1 and f1 for L1 as body location, be transmitted to the body-mountable medical device 2.

The steps of the user selecting a body location is typically part of an initialization routine that is carried out in the process of attaching the body-wearable medical device 1. Typical body-wearable medical devices, in particular continuous glucose measurement devices and insulin infusion systems, are normally carried by a Person with Diabetes (PwD) as patient substantially continuously, night and day. Maintenance actions, such as the replacement of a drug reservoir and/or the infusion cannula (for an insulin infusion pump), or the replacement of an electrochemical sensing element (for continuous a continuous glucose measurement device) need to be typically carried out in an interval of every few days up to every few weeks. As part of such maintenance action, the body location where the body-mountable medical device 1 is carried is changed by routine in order to minimize the undesired skin irritation resulting from the adhesive layer 32 contacting the skin, as well as the tissue irritation and potentially more severe complications resulting from the skin being pierced by transcutaneous element 16 for an extended time period at the same location. As part of such maintenance action, an initialization routine is generally carried out where, for example, various internal parameters of the medical system may be reset, a new sensing element is initialized, and the like.

**Figure 4** schematically illustrates various examples for providing tactile indications. In all examples, it is assumed that the tactile indicator 14 is activated by powering it with a control signal in form of a time-varying voltage U. Figure 4 (a) and Figure (b) both illustrate the indication of a first information that may correspond to an alarm situation that requires immediate attention, such as a device defect or a blocked occlusion cannula. In this example, a continuous indication shall be provided until the alarm is acknowledged by the user. Therefore, the tactile indicator 14 is continuously activated. Figure (a) illustrates the voltage U for a first body location, for example L1. Exemplarily, the voltage U is a square-form voltage with amplitude a1 and a period T1 = 1/f1 as control variables of the active control pattern. Figure 4 (b) illustrates the voltage U for the same type of indication as in Figure 4 (b), but for a different body location, for example L2. Exemplarily, the amplitude a2 is larger than the amplitude a1 and the period T2 = 1/f2 is larger than T1 (i. e. the frequency f2 is smaller than the frequency f1).

Figure 4 (c) and Figure (d) both illustrate the indication of a second information that may for example correspond to a user feedback upon a data input via user interface 22. In this example, the indication that shall be provided is given by a number of e. g. two vibration bursts that are separated by pause. Therefore, the tactile indicator 14 is activated in with two bursts B. Regarding the body location, the body location is identical for Figure 4 (c) and Figure4 (a), and is further identical for Figure 4 (d) and Figure 4 (b). Consequently, each burst corresponds to an activation of the tactile indicator 14 with amplitude a1 and period T1 (frequency f1) in Figure 4 (c), and an activation with amplitude a2 and period T2 (frequency f2) in Figure 4 (d). The activation of the tactile indicator 14 respectively the generation of the control signal is controlled by the medical device control unit 11.

It can be seen that, in this example, the control pattern (determined by frequency and amplitude) only depends on the body location (independent of the indication information), while the indication information determines the timing of activating and deactivating (toggling between "on" and "off") of the tactile indicator 14.

## Claims

1. Medical system, including:
a) a skin-mountable medical device (1), the skin-mountable medical device (1) including an activatable tactile indicator (14);
b) a user interface (22), the user interface (22) being configured to receive location information, the location information indicating a body location where the skin-mountable medical device (1) shall be mounted;
wherein the medical system is configured to determine an active control pattern in dependence of the location information and to control activation of the tactile indicator (14) in accordance with the active control pattern.

2. Medical system according to claim 1, wherein the activatable tactile indicator (14) is a vibrator.

3. Medical system according to any of the preceding claims, wherein the medical system comprises a set of predetermined control patterns and the active control pattern is selected from the set of predetermined control patterns.

4. Medical system according to any of the preceding claims, wherein the medical system includes a remote device (2), wherein the skin-mountable medical device (1) and the remote device (2) are configured for wireless data exchange with each other, wherein the user interface (22) is provided on the remote device (2).

5. Medical system according to claim 4, wherein the remote device (2) is configured to determine the active control pattern and the medical system is configured for transmitting information indicative of the active control pattern from the remote device (2) to the skin-mountable medical device (1).

6. Medical system according to claim 4 , wherein the medical system is configured for transmitting information indicative of the location information from the remote device (2) to the skin-mountable medical device (1) and the skin-mountable medical device (1) is configured to determine the active control pattern based on the retrieved information.

7. Medical system according to any of the preceding claims 3 to 6, wherein the activatable tactile indicator (14) is a vibrator and different control patterns among the set of predetermined control patterns for operating the activatable tactile indicator (14) differ in at least one of frequency, amplitude, number of activation bursts, sequences of activation bursts, and pauses between activation bursts.

8. Medical system according to any of the preceding claims, wherein the skin-mountable medical device (1) includes a continuous glucose-measurement device and/or an insulin infusion pump.

9. Medical system according to any of the preceding claims, wherein the skin-mountable medical device (1) is configured to control activation of the activatable tactile indicator (14) in accordance with an indication information that shall be indicated to the patient.

10. A remote device (2), including:
a user interface (22), wherein the user interface (22) is configured to receive location information, the location information indicating a body location where a skin-mountable medical device (1) including an activatable tactile indicator (14) shall be mounted;
wherein the remote device (2) is configured to determine an active control pattern in dependence of the location information and
i.) to control activation of the tactile indicator (14) of the skin-mountable medical device (1) in accordance with the active control pattern; and/or
ii.) to transmit information indicative of the control pattern from the remote device (2) to the skin-mountable device (1).

11. Method of providing tactile indications to a user of a medical system, wherein the medical system includes a skin-mountable medical device (1), the method including:
a) receiving, via a user interface (22) of the medical system, location information, the location information indicating a body location where the skin-mountable medical device (1) shall be mounted;
b) determining, by the medical system, an active control pattern in dependence of the location information;
c) controlling activation of an activatable tactile indicator (14) in accordance with the active control pattern.

12. Method according to claim 11, wherein the method includes selecting the active control pattern from a set of predetermined control patterns included by the medical system.

13. Method according to claims 11 or 12, wherein the method includes providing the user interface on a remote device (2).

14. Method according to any of claims 11 to 13, wherein the method includes controlling activation of the activatable tactile indicator (14) in accordance with an indication information that shall be indicated to the patient.

## Patentansprüche

1. Medizinisches System, das Folgendes einschließt:
a) eine auf der Haut befestigbare medizinische Vorrichtung (1), wobei die auf der Haut befestigbare medizinische Vorrichtung (1) einen aktivierbaren taktilen Indikator (14) einschließt;
b) eine Benutzerschnittstelle (22), wobei die Benutzerschnittstelle (22) dafür ausgebildet ist, Ortsinformationen zu empfangen, wobei die Ortsinformationen einen Ort am Körper angeben, an dem die auf der Haut befestigbare medizinische Vorrichtung (1) befestigt werden soll;
wobei das medizinische System dafür ausgebildet ist, ein aktives Steuerungsmuster in Abhängigkeit von den Ortsinformationen zu bestimmen und die Aktivierung des taktilen Indikators (14) gemäß dem aktiven Steuerungsmuster zu steuern.

2. Medizinisches System nach Anspruch 1, wobei der aktivierbare taktile Indikator (14) ein Vibrator ist.

3. Medizinisches System nach einem der vorstehenden Ansprüche, wobei das medizinische System einen Satz vorbestimmter Steuerungsmuster umfasst und das aktive Steuerungsmuster aus dem Satz vorbestimmter Steuerungsmuster ausgewählt ist.

4. Medizinisches System nach einem der vorstehenden Ansprüche, wobei das medizinische System eine Fernbedienungsvorrichtung (2) einschließt, wobei die auf der Haut befestigbare medizinische Vorrichtung (1) und die Fernbedienungsvorrichtung (2) für drahtlosen Datenaustausch miteinander ausgebildet sind, wobei die Benutzerschnittstelle (22) an der Fernbedienungsvorrichtung (2) vorgesehen ist.

5. Medizinisches System nach Anspruch 4, wobei die Fernbedienungsvorrichtung (2) dafür ausgebildet ist, das aktive Steuerungsmuster zu bestimmen, und das medizinische System dafür ausgebildet ist, Informationen, die das aktive Steuerungsmuster angeben, von der Fernbedienungsvorrichtung (2) an die auf der Haut befestigbare medizinische Vorrichtung (1) zu übermitteln.

6. Medizinisches System nach Anspruch 4, wobei das medizinische System dafür ausgebildet ist, Informationen, die die Ortsinformationen angeben, von der Fernbedienungsvorrichtung (2) an die auf der Haut befestigbare medizinische Vorrichtung (1) zu übermitteln, und die auf der Haut befestigbare medizinische Vorrichtung (1) dafür ausgebildet ist, das aktive Steuerungsmuster basierend auf den erhaltenen Informationen zu bestimmen.

7. Medizinisches System nach einem der vorstehenden Ansprüche 3 bis 6, wobei der aktivierbare taktile Indikator (14) ein Vibrator ist und sich verschiedene Steuerungsmuster aus dem Satz vorbestimmter Steuerungsmuster zum Bedienen des aktivierbaren taktilen Indikators (14) zumindest hinsichtlich eines von Frequenz, Amplitude, Anzahl an Aktivierungsstößen, Abfolgen von Aktivierungsstößen und Pausen zwischen Aktivierungsstößen unterscheiden.

8. Medizinisches System nach einem der vorstehenden Ansprüche, wobei die auf der Haut befestigbare medizinische Vorrichtung (1) eine kontinuierliche Blutzuckermessvorrichtung und/oder eine Insulininfusionspumpe einschließt.

9. Medizinisches System nach einem der vorstehenden Ansprüche, wobei die auf der Haut befestigbare medizinische Vorrichtung (1) dafür ausgebildet ist, die Aktivierung des aktivierbaren taktilen Indikators (14) gemäß Anzeigeinformationen, die dem Patienten angezeigt werden sollen, zu steuern.

10. Fernbedienungsvorrichtung (2), die Folgendes einschließt:
eine Benutzerschnittstelle (22), wobei die Benutzerschnittstelle (22) dafür ausgebildet ist, Ortsinformationen zu empfangen, wobei die Ortsinformationen einen Ort am Körper angeben, an dem eine auf der Haut befestigbare medizinische Vorrichtung (1), die einen aktivierbaren taktilen Indikator (14) einschließt, befestigt werden soll;
wobei die Fernbedienungsvorrichtung (2) dafür ausgebildet ist, ein aktives Steuerungsmuster in Abhängigkeit von den Ortsinformationen zu bestimmen und
i.) die Aktivierung des taktilen Indikators (14) der auf der Haut befestigbaren medizinischen Vorrichtung (1) gemäß dem aktiven Steuerungsmuster zu steuern und/oder
ii.) Informationen, die das Steuerungsmuster angeben, von der Fernbedienungsvorrichtung (2) an die auf der Haut befestigbare Vorrichtung (1) zu übermitteln.

11. Verfahren zum Bereitstellen taktiler Anzeigen einem Benutzer eines medizinischen Systems, wobei das medizinische System eine auf der Haut befestigbare medizinische Vorrichtung (1) einschließt, wobei das Verfahren Folgendes einschließt:
a) Empfangen von Ortsinformationen über eine Benutzerschnittstelle (22) des medizinischen Systems, wobei die Ortsinformationen einen Ort am Körper angeben, an dem die auf der Haut befestigbare medizinische Vorrichtung (1) befestigt werden soll;
b) Bestimmen eines aktiven Steuerungsmusters in Abhängigkeit von den Ortsinformationen durch das medizinische System;
c) Steuern der Aktivierung eines aktivierbaren taktilen Indikators (14) gemäß dem aktiven Steuerungsmuster.

12. Verfahren nach Anspruch 11, wobei das Verfahren das Auswählen des aktiven Steuerungsmusters aus einem Satz vorbestimmter Steuerungsmuster, die in dem medizinischen System enthalten sind, einschließt.

13. Verfahren nach den Ansprüchen 11 oder 12, wobei das Verfahren das Bereitstellen der Benutzerschnittstelle auf einer Fernbedienungsvorrichtung (2) einschließt.

14. Verfahren nach einem der Ansprüche 11 bis 13, wobei das Verfahren das Steuern der Aktivierung des aktivierbaren taktilen Indikators (14) gemäß Anzeigeinformationen, die dem Patienten angezeigt werden sollen, einschließt.

## Revendications

1. Système médical, comprenant :
a) un dispositif médical monté sur la peau (1), le dispositif médical monté sur la peau (1) comprenant un indicateur tactile activable (14) ;
b) une interface utilisateur (22), l'interface utilisateur (22) étant configurée pour recevoir des informations de localisation, les informations de localisation indiquant une localisation sur le corps où le dispositif médical monté sur la peau (1) doit être monté ;
dans lequel le système médical est configuré pour déterminer un modèle de commande actif dépendant des informations de localisation et pour commander l'activation de l'indicateur tactile (14) conformément au modèle de commande actif.

2. Système médical selon la revendication 1, dans lequel l'indicateur tactile activable (14) est un vibreur.

3. Système médical selon l'une quelconque des revendications précédentes, dans lequel le système médical comprend un ensemble de modèles de commande prédéterminés et le modèle de commande actif est choisi depuis l'ensemble de modèles de commande prédéterminés.

4. Système médical selon l'une quelconque des revendications précédentes, dans lequel le système médical comprend un dispositif à distance (2), dans lequel le dispositif médical monté sur la peau (1) et le dispositif à distance (2) sont configurés pour un échange de données sans fil l'un avec l'autre, dans lequel l'interface utilisateur (22) est pourvue sur le dispositif à distance (2).

5. Système médical selon la revendication 4, dans lequel le dispositif à distance (2) est configuré pour déterminer le modèle de commande actif et le système médical est configuré pour transmettre des informations indicatives du modèle de commande actif du dispositif à distance (2) au dispositif médical monté sur la peau (1).

6. Système médical selon la revendication 4, dans lequel le système médical est configuré pour transmettre des informations indicatives des informations de localisation du dispositif à distance (2) au dispositif médical monté sur la peau (1) et le dispositif médical monté sur la peau (1) est configuré pour déterminer le modèle de commande actif à partir des informations récupérées.

7. Système médical selon l'une quelconque des revendications 3 à 6 précédentes, dans lequel l'indicateur tactile activable (14) est un vibreur et différents modèles de commande parmi l'ensemble de modèles de commande prédéterminés pour le fonctionnement de l'indicateur tactile activable (14) diffèrent d'au moins un parmi la fréquence, l'amplitude, le nombre d'impulsions d'activation, les séquences d'impulsions d'activation et les pauses entre les impulsions d'activation.

8. Système médical selon l'une quelconque des revendications précédentes, dans lequel le dispositif médical monté sur la peau (1) comprend un dispositif de mesure de glycémie en continu et/ou une pompe de perfusion d'insuline.

9. Système médical selon l'une quelconque des revendications précédentes, dans lequel le dispositif médical monté sur la peau (1) est configuré pour commander l'activation de l'indicateur tactile activable (14) conformément à une information d'indication qui doit être indiquée au patient.

10. Dispositif à distance (2), comprenant :
une interface utilisateur (22), dans lequel l'interface utilisateur (22) est configurée pour recevoir des informations de localisation, les informations de localisation indiquant une localisation sur le corps où un dispositif médical monté sur la peau (1) comprenant un indicateur tactile activable (14) doit être monté ;
dans lequel le dispositif à distance (2) est configuré pour déterminer un modèle de commande actif dépendant des informations de localisation et
i.) pour commander l'activation de l'indicateur tactile (14) du dispositif médical monté sur la peau (1) conformément au modèle de commande actif ; et/ou
ii.) pour transmettre des informations indiquant le modèle de commande du dispositif à distance (2) au dispositif monté sur la peau (1).

11. Procédé de fourniture d'indications tactiles à un utilisateur d'un système médical, dans lequel le système médical comprend un dispositif médical monté sur la peau (1), le procédé comprenant :
a) la réception, via une interface utilisateur (22) du système médical, d'informations de localisation, les informations de localisation indiquant une localisation sur le corps où le dispositif médical monté sur la peau (1) doit être monté ;
b) la détermination, par le système médical, d'un modèle de commande actif dépendant des informations de localisation ;
c) la commande de l'activation d'un indicateur tactile activable (14) conformément au modèle de commande actif.

12. Procédé selon la revendication 11, dans lequel le procédé comprend la sélection du modèle de commande actif depuis un ensemble de modèles de commande prédéterminés compris par le système médical.

13. Procédé selon les revendications 11 ou 12, dans lequel le procédé comprend la fourniture de l'interface utilisateur sur un dispositif à distance (2).

14. Procédé selon l'une quelconque des revendications 11 à 13, dans lequel le procédé comprend la commande de l'activation de l'indicateur tactile activable (14) conformément à une information d'indication qui doit être indiquée au patient.
